(19) Europäisches Patentamt
European Patent Office
Office européen des brevets

(11) **EP 1 572 329 B1**

(12) **EUROPÄISCHE PATENTSCHRIFT**

(45) Veröffentlichungstag und Bekanntmachung des Hinweises auf die Patenterteilung:
**11.08.2010 Patentblatt 2010/32**

(21) Anmeldenummer: **03779905.3**

(22) Anmeldetag: **13.11.2003**

(51) Int Cl.:
**B01D 61/42** (2006.01)     **B01D 57/02** (2006.01)
**G01N 27/447** (2006.01)     **C07K 1/26** (2006.01)

(86) Internationale Anmeldenummer:
**PCT/EP2003/012665**

(87) Internationale Veröffentlichungsnummer:
**WO 2004/045748 (03.06.2004 Gazette 2004/23)**

(54) **VORRICHTUNG UND VERFAHREN ZUR PRÄPARATIVEN ELEKTROPHORESE**

DEVICE AND METHOD FOR PREPARATORY ELECTROPHORESIS

DISPOSITIF ET PROCEDE D'ELECTROPHORESE PREPARATOIRE

(84) Benannte Vertragsstaaten:
**AT BE BG CH CY CZ DE DK EE ES FI FR GB GR HU IE IT LI LU MC NL PT RO SE SI SK TR**

(30) Priorität: **18.11.2002 DE 10253483**

(43) Veröffentlichungstag der Anmeldung:
**14.09.2005 Patentblatt 2005/37**

(73) Patentinhaber: **Bayer Technology Services GmbH**
**51368 Leverkusen (DE)**

(72) Erfinder:
• **DUDZIAK, Gregor**
**Oakland, CA 94602 (US)**

• **NICKEL, Andreas**
**58300 Wetter (DE)**
• **MUTTER, Martina**
**51063 Köln (DE)**
• **BAUMARTH, Kerstin**
**42277 Wuppertal (DE)**

(56) Entgegenhaltungen:
EP-A- 0 369 945     WO-A-94/11728
US-A- 4 043 895     US-A- 4 043 896
US-A- 4 180 451     US-A- 4 758 320

**Beschreibung**

Die Erfindung betrifft ein Verfahren und eine Vorrichtung zur Durchführung der Membranelektrophorese mittels Mikro- oder Ultrafiltrationsmembranen. Dabei wird der elektroosmotische Fluss, der sich in den Membranporen ausbildet, durch Drucküberlagerung kompensiert.

Der Einsatz der Elektrophorese als präparative Trenntechnik wird seit den 50er Jahren untersucht.

Bei der kontinuierlichen Freifluss-Elektrophorese strömt eine Lösung in eine Elektrophoresekammer ein und wird zwischen zwei Elektroden im elektrischen Feld separiert. Beim Austritt aus dem Elektrophoresemodul wird der Flüssigkeitsstrom in eine Vielzahl von Fraktionen aufgeteilt, die die zu trennenden Substanzen in unterschiedlichen Konzentrationen enthalten. Obwohl diese Technik im Labormaßstab zu guten Selektivitäten führt, ist eine Maßstabsvergrößerung nur in engen Grenzen möglich. Als Hauptproblem gilt die Erwärmung der Lösung im elektrischen Feld sowie die daraus resultierenden Dispersionsphänomene wie etwa Wärmekonvektion. Mit kommerziell erhältlichen Freifluss-Elektrophorese-Geräten können Produktivitäten von einigen Gramm Produkt pro Tag erzielt werden.

Bei der Membranelektrophorese wirken semipermeable Membranen als Konvektionsbarrieren zwischen angrenzenden Kompartimenten, wobei mindestens eine gelöste Komponente im elektrischen Feld von einem Kompartiment in ein anderes wandern kann.

In ersten Veröffentlichungen wurden makroporöse Membranen, zum Beispiel Filterpapier, eingesetzt. Diese Materialien haben jedoch eine Reihe von Nachteilen: Sie weisen keinerlei Selektivität für die zu trennenden Substanzen auf (US-A-3 989 613, US-A-4 043 895) und haben darüber hinaus oft nur eine geringe mechanische und chemische Stabilität. Überdies zeigen sie bereits bei Druckdifferenzen von weniger als 1 kPa nicht zu vernachlässigende druckinduzierte Transmembranflüsse. Zeitlich oder örtlich variable Druckdifferenzen zwischen einzelnen Kompartimenten führen somit zu einer Rückvermischung und einer Verminderung der Trennleistung. Dieser druckinduzierte Permeatfluss wurde von Gritzner durch Verschaltung mit Ausgleichstanks verringert (US-A-4 043 895). Durch variable Füllstände regulieren sich die hydrostatischen Drücke in den beiden Strömungskanälen selbständig auf denselben Wert ein.

Spätere Patente schlagen den Einsatz von Ultrafiltrationsmembranen vor, die eine Selektivität für Makromoleküle unterschiedlicher Größe aufweisen. Die Trennleistung kann somit in der Theorie durch die Kombination der Selektivitätskriterien elektrophoretische Mobilität und Membranrückhalt deutlich verbessert werden. Außer Offenlegungsschriften ohne Ausführungs-Beispiel (DE 3 337 669 A1, DE 3 626 953 A1) wurden Batch-Versuche im Milliliter-Maßstab veröffentlicht (US-A-6 270 672).

In der Praxis wird beim Einsatz von Ultrafiltrationsmembranen jedoch eine sehr geringe Produktivität beobachtet. In den Membranporen bildet sich eine elektrische Doppelschicht aus, was im elektrischen Feld zur Induktion eines elektroosmotischen Flusses führt, der die Trennkapazität negativ geladener Proteine drastisch reduziert (Galier et al., J. Membr. Sci. 194 [2001] 117-133).

Ist eine Protein-Spezies unter Separationsbedingungen positiv geladen, so kann die Trennung unter umgekehrter Elektroden-Polarität durchgeführt werden. In diesem Fall kann der elektroosmotische Fluss dem Proteintransport durch die Membran entgegengerichtet oder gleichgerichtet sein. Dementsprechend beobachtet man ein Ansteigen bzw. ein Absinken des Flüssigkeitsstands im Diluat-Behälter. Während im ersten Fall eine Verminderung der Produktivität erfolgt, hat der zweite Fall eine Verringerung der Trennleistung zur Folge, da Proteine mit niedriger Mobilität, die im Diluat verbleiben sollen, konvektiv durch die Membran transportiert werden können.

Durch den Ausgleich hydrostatischer Druckunterschiede im Modul, wie etwa in US-A-4 043 895 beschrieben, können Flüssigkeitsströme durch die Separationsmembranen also nicht ausgeglichen werden.

Eine Alternative zum Einsatz poröser Ultra- und Mikrofiltrationsmembranen stellt die Verwendung von Gelmembranen dar. Dieses nichtporöse Material bietet den Vorteil eines geringen elektroosmotischen Flusses. Die Selektivität der Gelmembran für Makromoleküle kann durch den Grad der Polymer-Quervernetzung beeinflusst werden (vgl. US-A-4 243 507).

Gelmembranen weisen jedoch auch eine Reihe von Nachteilen gegenüber porösen Membranen auf:

Sie besitzen einen hohen Widerstand im elektrischen Feld. Dies führt zu einem hohen Energieeintrag und damit zu starker Wärmeentwicklung im Modul.

Gele wie Polyacrylamid besitzen eine schlechte pH-Stabilität und lassen sich daher nicht wie herkömmliche Ultra- und Mikrofiltrationsmembranen reinigen. Hieraus ergeben sich sehr hohe Kosten für den Modulersatz, da die Reinigung bei Bearbeitung von Proteinen essentiell ist.

Einerseits ist der Einsatz von Ultra- und Mikrofiltrationsmembranen für die Membranelektrophorese bisher durch den elektroosmotischen Effekt limitiert. Andererseits können Gelmembranen zwar mit den genannten Nachteilen im Labormaßstab eingesetzt werden, eine Maßstabsvergrößerung scheitert jedoch an zu hohen Membrankosten und Energieeintrag.

Der Erfindung liegt die Aufgabe zugrunde, ein verbessertes Membranelektrophorese-Verfahren unter Einsatz von Ultra- und Mikrofiltrationsmembranen zu entwickeln, das die hier genannten Nachteile nicht aufweist.

Aufgrund des elektroosmotischen Flusses, der sich in porösen Membranen ausbildet, war z.B. ein Einsatz im technischen Maßstab (>0,5 kg/h) bisher nicht möglich.

Es wurde gefunden, dass der elektroosmotische Fluss in den verwendeten Membranen und die daraus resultierenden Volumenänderungen durch Überlagerung eines geregelten hydrostatischen Druckes kompensiert werden können.

Gegenstand der Erfindung ist ein Verfahren zur Membranelektrophorese von gelösten oder dispergierten Substanzen in elektrolythaltiger Lösung gemäß Anspruch 1 unter Verwendung einer Trennkammer, die Diluaträume, Konzentraträume, sowie einen Kathodenraum und einen Anodenraum mit Elektroden als Anode bzw. Kathode aufweist, wobei die einzelnen Räume durch poröse Membranen, insbesondere Ultra- oder Mikrofiltrationsmembranen voneinander getrennt sind und die Elektroden von Elektrodenspüllösung umspült werden und das Diluat kontinuierlich durch den Diluatraum, bzw. das Konzentrat kontinuierlich durch den Konzentratraum geführt werden, **dadurch gekennzeichnet, dass** mindestens eine im Diluat gelöste oder dispergierte Substanz mittels eines zwischen Anode und Kathode angelegten elektrischen Feldes elektrophoretisch vom Diluatraum zum Konzentratraum überführt wird, wobei zwischen dem Diluatraum und dem Konzentratraum eine Druckdifferenz von mindestens 3 kPa so eingestellt wird, dass ein Flüssigkeitsstrom durch die Separationsmembran, die Konzentratraum und Diluatraum voneinander trennt, im Wesentlichen unterbunden wird.

Das Verfahren wird in einer Trennkammer durchgeführt, die aus jeweils mehreren Diluaträumen und Konzentraträumen besteht, wobei die Diluaträume und Konzentraträume zwischen Anodenraum und Kathodenraum alternierend angeordnet sind, die durch Ultra- und/oder Mikrofiltrations-Membranen voneinander getrennt sind und parallel und/oder in Reihe verbunden betrieben werden.

Vorteilhafterweise werden in einer bevorzugten Ausführung Diluatflüssigkeit, Konzentratflüssigkeit und Elektrodenspüllösung oder einzelne dieser Lösungen unabhängig voneinander temperiert, bevorzugt gekühlt.

Die porösen Membranen weisen insbesondere eine Porengröße von 1 bis 1000 nm auf.

Bevorzugt basieren die Membranen auf einem oder mehreren der folgenden Werkstoffe:

Celluloseester, Polyacrylnitril, Polyamid, Polyether, Polyethersulfon, Polypropylen, Polysulfon, Polyvinylalkohol, Polyvinylidenfluorid oder Aluminiumoxid, Siliciumoxid, Titanoxid, Zirkonoxid sowie Mischkeramiken aus den oben genannten Oxiden.

Besonders bevorzugt ist ein Verfahren, bei dem Anodenraum und Kathodenraum unabhängig voneinander mit Elektrodenspüllösung durchspült werden.

Die für die Diluatlösung, die Konzentratlösung und Elektrodenspüllösung verwendeten Elektrolyte enthalten bevorzugt eine Kombination aus schwachen Säuren und schwachen Basen, schwachen Säuren und starken Basen oder starken Säuren und schwachen Basen.

Besonders bevorzugt enthalten die Elektrolyte eine oder mehrere der folgenden Verbindungen:

Borsäure, Phosphorsäure, N-2-(Acetamido)-2-aminoethansulfonsäure, N-2-(Acetamido)iminodiessigsäure, Alanin, 2-Amino-2-methyl-1,3-propandiol, Ammoniak, N,N-Bis(2-hydroxyethyl)-2-aminoethansulfonsäure, N,N-Bis(2-hydroxyethyl)glycin, 2,2-Bis(hydroxyethyl)-iminotris(hydroxymethyl)methan, 2-(Cyclohexylamino)ethansulfonsäure, Essigsäure, Glycin, Glycylglycin, 2-[4-(2-Hydroxyethyl)1-piperazinyl]ethansulfonsäure (HEPES), 3-[4-(2-Hydroxyethyl)1-piperazinyl]propansulfonsäure, Histidin, Imidazol, Milchsäure, 2-Morpholinoethansulfonsäure (MES), 2-Morpholinopropansulfonsäure, Piperazin-1,4-bis(2-ethansulfonsäure), N-[Tris(hydroxymethyl)-methyl]-2-aminoethansulfonsäure, N-[Tris(hydroxymethyl)-methyl]glycin, Triethanolamin, Tris(hydroxymethyl)aminomethan, Zitronensäure.

Die Stromdichte bezogen auf die Membranfläche beträgt bevorzugt 10 bis 1000 A/m$^2$, insbesondere 10 bis 500 A/m$^2$.

Die Leitfähigkeit der Diluatlösung beträgt bevorzugt 0,1 mS/cm bis 40 mS/cm, insbesondere 0,1 bis 10 mS/cm.

Bevorzugt ist auch ein Verfahren bei dem die Leitfähigkeit der Diluatlösung während der Trennung abgesenkt wird.

Vorteilhaft ist weiterhin ein Verfahren, **dadurch gekennzeichnet, dass** die Diluatlösung im Anschluss an die Trennung aufkonzentriert wird, insbesondere durch Kombination mit einer Flüssigpermeation, wie zum Beispiel Mikrofiltration, Ultrafiltration, Nanofiltration oder Umkehrosmose, und dem Diluatraum wieder zugeführt wird.

Dem Verfahren zugänglich sind insbesondere eine oder mehrere der folgenden Substanzen: Proteine, Peptide, DNA, RNA, Oligonucleotide, Oligo- und Polysaccharide, Viren, Virenbestandteile, Zellen, Zellenbestandteile, Enantiomere und Diastereomere.

Weiterer Gegenstand der Erfindung ist eine Vorrichtung zur Membranelektrophorese gemäß Anspruch 13 mindestens umfassend eine Trennkammer, die mindestens Diluaträume, Konzentraträume, sowie einen Kathodenraum und einen Anodenraum mit Elektroden als Anode bzw. Kathode aufweist, wobei die einzelnen Räume durch poröse Membranen, insbesondere durch Ultra- oder Mikrofiltrationsmembranen voneinander getrennt sind, Zuleitungen und Ableitungen für das Diluat, Zuleitungen und Ableitungen für das Konzentrat, ggf. Zuleitungen und Ableitungen für die Elektrodenspüllösung sowie einer Einrichtung zur Druckregelung, mittels der zwischen Diluatraum und Konzentratraum eine Druckdifferenz insbesondere von mindestens 3 kPa erzeugbar ist.

Die Trennkammer ist in mehrere Diluaträume und Konzentraträume unterteilt.

Zwischen Anodenraum und Kathodenraum sind bevorzugt alternierend mehrere Diluaträume und Konzenträume angeordnet, die durch poröse Restriktionsmembranen bzw. Separationsmembranen voneinander getrennt sind und die bevorzugt parallel und/oder in Reihe geschaltet sind.

Bevorzugt ist weiter eine Vorrichtung, in der Zuleitungen und Ableitungen für das Diluat in einem Diluatkreislauf, Zuleitungen und Ableitungen für das Konzentrat in einem Konzentratkreislauf, ggf. Zuleitungen und Ableitungen für die Elektrodenspüllösung in einem Elektrodenspülungskreislauf angeordnet sind.

Bevorzugt ist ebenfalls eine Vorrichtung, die einen Diluatkreislauf, Konzentratkreislauf und Elektrodenspülungskreislauf aufweist und insbesondere Wärmetauscher in einzelnen oder allen dieser Kreisläufe aufweist.

Der Elektrodenspülungskreislauf ist in einer bevorzugten Variante durch einen getrennten Anodenspülkreislauf und Kathodenspülkreislauf gebildet.

Der elektroosmotische Fluss wird durch Drucküberlagerung in einzelnen druckdichten Vorlagegefäßen kompensiert. Die Druckdifferenz kann bis einige 100 kPa betragen.

Die Vorrichtung eignet sich zur Aufreinigung gelöster oder disperser Substanzen in wässrigem Medium. Anwendungsbeispiele sind die Reinigung von Proteinen, Peptiden, DNA, RNA, Oligonucleotiden, Viren, Zellen sowie chiraler Moleküle.

Besonders geeignet ist die Methode zur Aufreinigung von Proteinen, Peptiden, Oligonukleotiden und Viruspartikeln.

Gegenstand der Erfindung ist daher auch die Verwendung der erfindungsgemäßen Vorrichtung zur Reinigung von Proteinen, Peptiden, DNA, RNA, Oligonucleotiden, Viren, Zellen oder chiralen Molekülen.

Die Erfindung kann sowohl im Batch-Betrieb als auch bei kontinuierlichem Betrieb angewendet werden.

Die Erfindung wird nachfolgend anhand der Figuren durch die Beispiele, welche jedoch keine Beschränkung der Erfindung darstellen, näher erläutert.

Es zeigen:

Fig. 1     das Schema einer Membranelektrophorese-Anlage mit Einrichtung zur Drucküber- lagerung

Fig. 2     ein Schema eines Membranelektrophorese-Moduls (Vierer-Stack)

## Beispiele

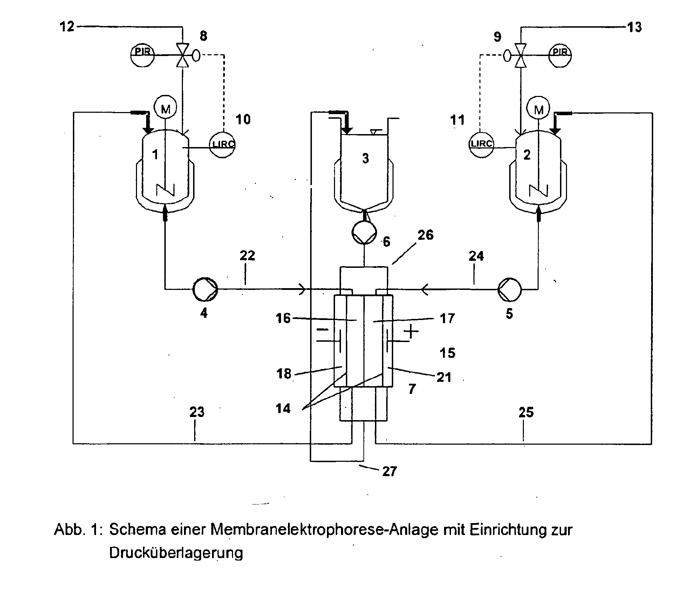

Die bei den nachfolgend beschriebenen Beispielen verwendete Anlage (Figur 1) besteht aus je einem temperierbaren Vorlagebehälter für Diluat 1, Konzentrat 2 und Elektrodenpuffer 3. Die Lösungen werden mittels Pumpen 4, 5, 6 über Vorlaufleitungen 22, 24, 26 und Rücklaufleitungen 23, 25, 27 rezirkuliert und durchströmen ein Elektrophoresetrennmodul 7. Die Drucküberlagerung im Gasraum des Diluat- und des Konzentrat-Behälters mit Stickstoff aus den Leitungen 12, 13 erfolgt mittels Nachdruckregler 8, 9. Die Nachdruckregler werden wiederum über Füllstandssonden 10, 11 geregelt.

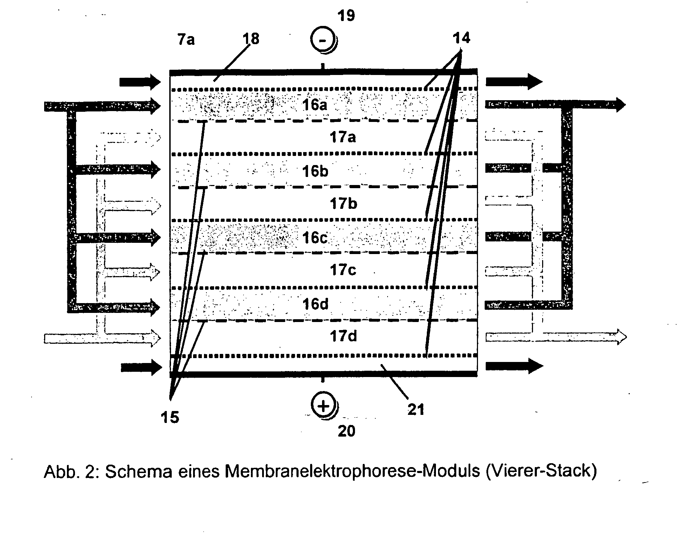

Die Membranelektrophorese-Anlage enthält ein Modul 7a (vergleiche Fig. 2) mit jeweils vier parallelen Diluaträumen 16a-d und Konzenträumen 17a-d. Die Diluat- 16 und Konzenträume 17 werden über Flüssigkeitsverteiler 28, 29 parallel angeströmt und durch Restriktionsmembranen 14 und Separationsmembranen 15 begrenzt. Diluat- und Konzenträume können hier nicht gezeigte Gitternetze oder Gewebe enthalten, die als Abstandhalter zwischen den Membranen und als Strömungsbrecher fungieren. Die Elektrodenräume 18, 21 werden parallel angeströmt und durch Restriktionsmembranen 14 begrenzt. Über Elektroden 19, 20 wird ein elektrisches Feld aufgebaut. Das elektrische Feld kann wie in Figur 2 eingezeichnet oder auch entgegengesetzt aufgebaut werden.

## Beispiel 1:

Die in Figur 1 gezeigte Anlage sowie das in Figur 2 skizzierte Modul 7a wurden zur Trennung von humanem Serumalbumin (HSA) und humanem Immunoglobulin G (IgG) eingesetzt. Bei dem Modul 7a handelte es sich um ein modifiziertes Elektrodialysemodul (Elektrodialysemodul ED 136; FuMA-Tech GmbH) mit einer effektiven Membranfläche von 36 cm$^2$ pro Membranlage.

Es wurde ein HEPES (2-[4-(2-hydroxyethyl)-1-piperazinyl]ethansulfonsäure)/Imidazol-Puffer verwendet (ca. 40 mM HEPES/15 mM Imidazol, pH 7). Die Vorlagen für Konzentratlösung 2 und Elektrodenspüllösung 3 wurden mit jeweils 1000 mL Pufferlösung befüllt. Die Diluat-Vorlage wurde mit 400 mL im Puffer gelöstem HSA mit einer Konzentration von 38 g/L sowie IgG in einer Konzentration von 4,5 g/L befüllt.

Restriktionsmembranen 14 mit einer nominalen Trenngrenze von 10 kDa sowie Separationsmembranen 15 mit einer nominalen Trenngrenze von 300 kDa wurden mit Standard-Spacem (Hersteller:FuMA-Tech GmbH) zu einem Vierfach-Stack zusammengefügt. Bei den verwendeten Membranen handelte es sich um PES-Ultrafiltrationsmembranen der Firma Sartorius AG.

Der Versuch wurde bei einer Stromdichte von etwa 45 A/m$^2$ durchgeführt, wobei an die diluatseitige Elektrode 20 ein negatives Potential angelegt wurde. Die Volumenströme im Diluat- und Konzentratkreislauf betrugen jeweils 320 mL/min, der Volumenstrom im Elektrodenkreislauf 700 mL/min. Die Proteinkonzentrationen wurden durch HPLC-Analytik bestimmt.

Folgende Versuche wurden durchgeführt:

1a) Elektrophorese mit Kompensation des elektroosmotischen Flusses durch Regelung des Füllstands der Diluatvorlage mittels Druckbeaufschlagung

1b) Elektrophorese ohne Kompensation des elektroosmotischen Flusses Tabelle 1 enthält Versuchsparameter und Konzentrationsverläufe des Versuchs 1a, Tabelle 2 die Daten des Versuchs 1b. Versuch 1b musste nach 180 Minuten abgebrochen werden, da die Diluatvorlage vollgelaufen war.

Die Abnahme der Albumin-Konzentration im Diluat ist konzentrationsabhängig und folgt einer Kinetik erster Ordnung der Form:

$$\frac{dc}{dt} \cdot \frac{V}{A} = - k \cdot c$$

mit

| | | |
|---|---|---|
| c: | Albumin-Konzentration im Diluat | [g/L$^*$] |
| t: | Zeit | [h] |
| V: | Volumen Diluat | [L] |
| A: | Effektive Fläche der Separationsmembranen | [m$^2$] |
| k: | Geschwindigkeitskonstante | [L/(h m$^2$)] |

Durch Integration und Auflösung nach dem Restanteil erhält man:

$$\ln\left(\frac{c}{c_0}\right) \cdot \frac{V}{A} = - k \cdot t$$

mit

c/c$_0$:     Restanteil    [-]

Berücksichtigt man, dass das Diluat-Volumen nicht unbedingt konstant bleibt, so kann man durch Einsetzen der Proteinmassen anstelle der Konzentrationen eine effektive Geschwindigkeitskonstante berechnen:

$$\ln\left(\frac{m}{m_0}\right) \cdot \frac{V}{A} = - k_{eff} \cdot t$$

mit

| | | |
|---|---|---|
| m: | Albumin-Masse im Diluat | [g] |
| m$_0$: | Albumin-Masse im Diluat zu Versuchsbeginn | [g] |
| m/m$_0$: | massenbezogener Restanteil | [-] |
| k$_{eff}$: | effektive Geschwindigkeitskonstante | [L/(h m$^2$)] |

Für die Abreicherung von Serumalbumin ergeben sich nach 180 Minuten massenbezogene Restanteile von 0,45 (Versuch 1a) beziehungsweise 0,64 (Versuch 1b). Setzt man diese in die oben beschriebene Formel ein, so ergibt sich für Beispiel 1a im Vergleich zu Beispiel 1b eine 1,8fache effektive Geschwindigkeitskonstante. Die Abreicherung erfolgt bei Druckkompensation also annähernd doppelt so schnell.

Die Selektivität der Abreicherung berechnet sich wie folgt:

$$\Psi = \frac{\ln\left[m/m_0\ (HSA)\right]}{\ln\left[m/m_0\ (IgG)\right]}$$

mit

| | | |
|---|---|---|
| $\psi$: | Selektivität | [-] |
| $m/m_0$ (HSA): | massenbezogener Restanteil des HSA im Diluat | [-] |
| $m/m_0$ (IgG): | massenbezogener Restanteil des IgG im Diluat | [-] |

Über den gesamten Verlauf von Versuch 1a ergibt sich eine Selektivität von 8,8, während bei Versuch 1b lediglich eine Selektivität von 3,8 erzielt werden konnte.

**Beispiel 2:**

Die in Figur 1 gezeigte Anlage sowie das in Figur 2 skizzierte Modul 7a wurden für die Trennung von humanem Serumalbumin und Hämoglobin eingesetzt. Bei dem Modul 7a handelte es sich um ein modifiziertes Elektrodialysemodul wie in Beispiel 1 mit einer effektiven Membranfläche von 36 cm$^2$ pro Membranlage.

Es wurde ein 50 millimolarer MES/Histidin-Puffer verwendet (ca. 15 mM MES/35 mM Histidin, pH 6,5). Die Vorlagen für Konzentratlösung 2 und Elektrodenspüllösung 3 wurden mit 1 L beziehungsweise 800 mL Pufferlösung befüllt. Die Diluat-Vorlage 1 enthielt die beiden im Puffer gelösten Proteine mit Massenkonzentrationen von 4,5 g/L humanem Serumalbumin und 0,85 g/L Hämoglobin. Das Volumen der Diluat-Flüssigkeit betrug 400 mL und wurde während des Versuchs mittels Drucküberlagerung konstant gehalten.

Restriktionsmembranen 14 mit einer nominalen Trenngrenze von 10 kDa sowie Separationsmembranen 15 mit einer nominalen Trenngrenze von 300 kDa wurden mit Standard-Spacem wie in Beispiel 1 zu einem Vierfach-Stack zusammengefügt. Bei den verwendeten Membranen handelte es sich um PES-Ultrafiltrationsmembranen der Firma Sartorius AG.

Der Versuch wurde bei einer Stromdichte von 45 A/m$^2$ durchgeführt, wobei an die diluatseitige Elektrode 20 ein negatives Potential angelegt wurde. Die Volumenströme im Diluat- und Konzentratkreislauf betrugen jeweils 160 mL/min, der Volumenstrom im Elektrodenkreislauf 770 mL/min. Die Proteinkonzentrationen wurden durch HPLC-Analytik bestimmt. Die Füllstände in den Vorlagegefäßen wurden durch eine konzentratseitige Druckregelung konstant gehalten.

Nach 3 h wurde eine 92 %ige Abreicherung des humanen Serumalbumins im Diluat bei 83 % Hämoglobin-Ausbeute im Diluat erzielt. Der Versuchsverlauf ist in Tabelle 3 abgebildet.

**Tabelle 1**: Ergebnisse HSA/IgG-Trennung (Beispiel 1a).

| Zeit / min | Gasdruck / kPa (Diluatraum) | Volumen / mL (Diluat) | Stromdichte / A/m$^2$ | m (HSA) / g (Diluat) | m (IgG) / g (Diluat) |
|---|---|---|---|---|---|
| 0 | 0 | 439 | 45 | 14,3 | 1,6 |
| 30 | 0 | 451 | 47 | 12,5 | 1,6 |
| 60 | 9 | 451 | 47 | 10,9 | 1,5 |
| 90 | 14 | 462 | 45 | 10,0 | 1,5 |
| 120 | 15 | 462 | 45 | 8,6 | 1,4 |
| 150 | 18 | 466 | 42 | 7,5 | 1,3 |
| 180 | 20 | 474 | 50 | 6,5 | 1,3 |
| 210 | 21 | 474 | 45 | 5,7 | 1,3 |
| 240 | 20 | 474 | 45 | 4,9 | 1,3 |
| 270 | 23 | 482 | 42 | 4,2 | 1,3 |
| 300 | 24 | 466 | 45 | 3,3 | 1,4 |

(fortgesetzt)

| Zeit / min | Gasdruck / kPa (Diluatraum) | Volumen / mL (Diluat) | Stromdichte / A/m$^2$ | m (HSA) / g (Diluat) | m (IgG) / g (Diluat) |
|---|---|---|---|---|---|
| 330 | 21 | 474 | 42 | 2,7 | 1,3 |
| 360 | 22 | 462 | 42 | 2,4 | 1,3 |

**Tabelle 2**: Ergebnisse HSA/IgG-Trennung (Beispiel 1b).

| Zeit / min | Gasdruck / kPa (Diluatraum) | Volumen / mL (Diluat) | Stromdichte / A/m$^2$ | m (HSA) / g (Diluat) | m (IgG) / g (Diluat) |
|---|---|---|---|---|---|
| 0 | 0 | 423 | 45 | 13,8 | 1,8 |
| 30 | 0 | 470 | 42 | 12,9 | 1,6 |
| 60 | 0 | 520 | 45 | 11,5 | 1,3 |
| 90 | 0 | 602 | 40 | 10,2 | 1,5 |
| 120 | 0 | 684 | 42 | 9,3 | 1,2 |
| 150 | 0 | 750 | 42 | 9,7 | 1,7 |
| 180 | 0 | 820 | 42 | 8,8 | 1,6 |

**Tabelle 3**: Ergebnisse HSA/Hämoglobin-Trennung (Beispiel 2)

| Zeit / min | Gasdruck / kPa (Diluatraum) | Volumen / mL (Diluat) | Stromdichte / A/m$^2$ | m (HSA) / g (Diluat) | m (Häm) / g (Diluat) |
|---|---|---|---|---|---|
| 0 | 0 | 420 | 45 | 1,85 | 0,35 |
| 30 | 4 | 420 | 45 | 1,29 | 0,34 |
| 60 | 6 | 420 | 47 | 0,79 | 0,32 |
| 90 | 3 | 420 | 45 | 0,48 | 0,31 |
| 120 | 5 | 420 | 45 | 0,30 | 0,30 |
| 150 | 3 | 420 | 45 | 0,20 | 0,29 |
| 180 | 7 | 420 | 42 | 0,14 | 0,29 |

**Patentansprüche**

1. Verfahren zur Membranelektrophorese von gelösten oder dispergierten Substanzen in elektrolythaltiger Lösung unter Verwendung einer Trennkammer (7a), die mehrere Diluaträume (16a, 16b,...), Konzentraträume (17a, 17b,...), sowie einen Kathodenraum (18) und einen Anodenraum (21) mit Elektroden als Anode (19) bzw. Kathode (20) aufweist, wobei Diluaträume (16a, 16b,...) und Konzenträume (17a, 17b,...) jeweils durch eine poröse Separationsmembran (15) voneinander getrennt sind und wobei Kathodenraum (18) und Anodenraum (21) von den Diluaträumen (16a, 16b,...) und Konzenträumen (17a, 17b,...), sowie Paare von Diluaträumen (16a, 16b,...) und Konzenträumen (17a, 17b,...) untereinander, durch poröse Restriktionsmembranen (14) getrennt sind und die Elektroden (19, 20) von Elektrodenspüllösung umspült werden und das Diluat kontinuierlich durch den Diluatraum (16), bzw. das Konzentrat kontinuierlich durch den Konzentratraum (17) geführt werden, **dadurch gekennzeichnet, dass** mindestens eine im Diluat gelöste oder dispergierte Substanz mittels eines zwischen Anode (19) und Kathode (20) angelegten elektrischen Feldes elektrophoretisch vom Diluatraum (16) zum Konzentratraum (17) überführt wird, wobei zwischen dem Diluatraum (16) und dem Konzentratraum (17) eine Druckdifferenz so eingestellt wird, dass ein Flüssigkeitsstrom durch die Separationsmembran (15) im Wesentlichen unterbunden wird und mindestens 3 kPa beträgt und wobei diese Druckdifferenz mittels Drucküberlagerung in den Vorlagegefässen (1, 2) geregelt wird.

2. Verfahren nach Anspruch 1, **dadurch gekennzeichnet, dass** die mehreren Diluaträumen (16a, 16b,...) und Konzenträumen (17a, 17b,...) parallel und/oder in Reihe verbunden betrieben werden.

3. Verfahren nach einem der vorhergehenden Ansprüche, **dadurch gekennzeichnet, dass** Diluatflüssigkeit, Konzentratflüssigkeit und Elektrodenspüllösung oder einzelne dieser Lösungen unabhängig voneinander temperiert, bevorzugt gekühlt werden.

4.  Verfahren nach einem der vorhergehenden Ansprüche, **dadurch gekennzeichnet, dass** die Membranen eine Porengröße von 1 bis 1000 nm aufweisen.

5.  Verfahren nach Anspruch 4, **dadurch gekennzeichnet, dass** die Membranen auf einem der folgenden Werkstoffe basieren: Celluloseester, Polyacrylnitril, Polyamid, Polyether, Polyethersulfon, Polypropylen, Polysulfon, Polyvinylalkohol, Polyvinylidenfluorid, oder Aluminiumoxid, Siliciumoxid, Titanoxid, Zirkonoxid sowie Mischkeramiken aus den oben genannten Oxiden.

6.  Verfahren nach einem der vorhergehenden Ansprüche, **dadurch gekennzeichnet, dass** Anodenraum (21) und Kathodenraum (18) unabhängig voneinander mit Elektrodenspüllösung durchspült werden.

7.  Verfahren nach einem der vorhergehenden Ansprüche, **dadurch gekennzeichnet, dass** die für die Diluatlösung, die Konzentratlösung und Elektrodenspüllösung verwendeten Elektrolyte eine Kombination aus schwachen Säuren und schwachen Basen, schwachen Säuren und starken Basen oder starken Säuren und schwachen Basen enthalten.

8.  Verfahren nach Anspruch 7, **dadurch gekennzeichnet, dass** die Elektrolyte eine oder mehrere der folgenden Verbindungen enthalten: Borsäure, Phosphorsäure, N-2-(Acetamido)-2-aminoethansulfonsäure, N-2-(Acetamido) iminodiessigsäure, Alanin, 2-Amino-2-methyl-1,3-propandiol, Ammoniak, N,N-Bis(2-hydroxyethyl)-2-aminoethansulfonsäure, N,N-Bis-(2-hydroxyethyl)glycin, 2,2-Bis(hydroxyethyl)-imintris(hydroxymethyl)methan, 2-(Cyclohexylamino)ethansulfonsäure, Essigsäure, Glycin, Glycylglycin, 2-[4-(2-Hydroxyethyl)1-piperazinyl]ethansulfonsäure, 3-[4-(2-Hydroxyethyl)1-piperazinyl]propansulfonsäure, Histidin, Imidazol, Milchsäure, 2-Morpholinoethansulfonsäure, 2-Morpholinopropansulfonsäure, Piperazin-1,4-bis(2-ethansulfonsäure), N-[Tris(hydroxymethyl)-methyl]-2-aminoethansulfonsäure, N-[Tris(hydroxymethyl)-methyl]glycin, Triethanolamin, Tris(hydroxymethyl)aminomethan, Zitronensäure.

9.  Verfahren nach einem der vorhergehenden Ansprüche, **dadurch gekennzeichnet, dass** die Stromdichte bezogen auf die Membranfläche 10 bis 1000 A/m$^2$, bevorzugt 10 bis 500 A/m$^2$ beträgt.

10. Verfahren nach einem der vorhergehenden Ansprüche, **dadurch gekennzeichnet, dass** die Leitfähigkeit der Diluatlösung 0,1 mS/cm bis 40 mS/cm, bevorzugt 0,1 bis 10 mS/cm, beträgt.

11. Verfahren nach einem der vorhergehenden Ansprüche, **dadurch gekennzeichnet, dass** die Diluatlösung im Anschluss an die Trennung aufkonzentriert wird, insbesondere durch Kombination mit einer Flüssigpermeation, wie zum Beispiel Mikrofiltration, Ultrafiltration, Nanofiltration oder Umkehrosmose und dem Diluatraum (16) wieder zugeführt wird.

12. Verfahren nach einem der vorhergehenden Ansprüche, bei dem eine oder mehrere der folgenden Substanzen behandelt werden: Proteine, Peptide, DNA, RNA, Oligonucleotide, Oligo- und Polysaccharide, Viren, Virenbestandteile, Zellen, Zellenbestandteile, Enantiomere und Diastereomere.

13. Vorrichtung zur Membranelektrophorese mindestens umfassend eine Trennkammer (7a), die mehrere Diluaträume (16a, 16b,...) und Konzenträume (17a, 17b,...) sowie einen Kathodenraum (18) und einen Anodenraum (21) mit Elektroden als Anode (19) bzw. Kathode (20) aufweist, wobei die Diluaträume (16a, 16b,...) und Konzenträume (17a, 17b,...) jeweils durch eine Separationsmembran (15) und wobei Kathodenraum (18) und Anodenraum (21), von den Diluaträumen (16a, 16b,...) und Konzenträumen (17a, 17b,...), sowie Paare von Diluaträume (16a, 16b,...) und Konzenträume (17a, 17b,...) untereinander, durch poröse Restriktionsmembranen (14) voneinander getrennt sind, Zuleitungen (22) und Ableitungen (23) für das Diluat, Zuleitungen (24) und Ableitungen (25) für das Konzentrat, ggf. Zuleitungen (26) und Ableitungen (27) für die Elektrodenspüllösung sowie eine Einrichtung (8; 10) bzw. (9; 11) zur Druckregelung, mittels der zwischen Diluaträumen (16a, 16b,...) und Konzenträumen (17a, 17b,...) eine Druck-differenz von mindestens 3 kPa erzeugbar ist und so eingestellt werden kann, dass ein Flüssigkeitsstrom durch die Separationsmembranen (15) im Wesentlichen unterbunden werden kann wobei als Einrichtung zur Druckregelung druckdichte Vorlagegefäße (1, 2) mit Mitteln zur Drucküberlagerung (12, 13) versehen sind.

14. Vorrichtung nach Anspruch 13, **dadurch gekennzeichnet, dass** die mehreren Diluaträume (16a, 16b,...) und Kon-zenträume (17a, 17b,...) parallel und/oder in Reihe geschaltet sind.

15. Vorrichtung nach einem der Ansprüche 13 oder 14, **dadurch gekennzeichnet, dass** Zuleitungen (22) und Ableitungen (23) für das Diluat in einem Diluatkreislauf (1; 4; 22; 23), Zuleitungen (24) und Ableitungen (25) für das Konzentrat in einem Konzentratkreislauf (2; 5; 24; 25), ggf. Zuleitungen (26) und Ableitungen (27) für die Elektrodenspüllösung in einem Elektrodenspülungskreislauf (3; 6; 26; 27) angeordnet sind.

16. Vorrichtung nach einem der Ansprüche 13 bis 15, **dadurch gekennzeichnet, dass** sie einen Diluatkreislauf, Konzentratkreislauf und Elektrodenspülungskreislauf aufweist und Wärmetauscher in einzelnen oder allen dieser Kreisläufe aufweist.

17. Vorrichtung nach einem der Ansprüche 13 bis 16, **dadurch gekennzeichnet, dass** poröse Membranen mit einer Porengröße von 1 bis 1000 nm vorgesehen sind. Diese sind bevorzugt aus organischen oder anorganischen Materialien oder einer Mischung aus beiden hergestellt.

18. Vorrichtung nach einem der Ansprüche 13 bis 17, **dadurch gekennzeichnet, dass** die Membranen auf einem der Werkstoffe basieren, ausgewählt aus der Reihe: Celluloseester, Polyacrylnitril, Polyamid, Polyether, Polyethersulfon, Polypropylen, Polysulfon, Polyvinylalkohol, Polyvinylidenfluorid, oder aus Aluminiumoxid, Siliciumoxid, Titanoxid, Zirkonoxid sowie Mischkeramiken aus den oben genannten Oxiden.

19. Vorrichtung nach einem der Ansprüche 15 bis 18, **dadurch gekennzeichnet, dass** der Elektrodenspülungskreislauf durch einen getrennten Anodenspülkreislauf und Kathodenspülkreislauf gebildet ist.

## Claims

1. Method for the membrane electrophoresis of substances which are dissolved or dispersed in electrolyte-containing solution using a separation chamber (7a) which possesses two or more diluate spaces (16a, 16b, ...), concentrate spaces (17a, 17b, ...), and also a cathode space (18) and an anode space (21) having electrodes as anode (19) and cathode (20), with diluate spaces (16a, 16b, ...) and concentrate spaces (17a, 17b, ...) in each case being separated from each other by means of a porous separation membrane (15) and with cathode space (18) and anode space (21) being separated from the diluate spaces (16a, 16b, ...) and concentrate spaces (17a, 17b, ...) and also pairs of diluate spaces (16a, 16b, ...) and concentrate spaces (17a, 17b, ...) being separated within each other by means of porous restriction membranes (14) and the electrodes (19, 20) being circulated by electrode washing solution and the diluate being continuously conducted through the diluate space (16), and, respectively, the concentrate being continuously conducted through the concentrate space (17), **characterized in that** at least one substance which is dissolved or dispersed in the diluate is transferred electrophoretically, by means of an electrical field which is applied between the anode (19) and the cathode (20), from the diluate space (16) to the concentrate space (17), with a pressure difference being established between the diluate space (16) and the concentrate space (17) such that any liquid flow through the separation membrane (15) is essentially prevented and being at least 3 kPa and with this pressure difference being regulated by means of pressure superposition in the vessels (1, 2).

2. Method according to Claim 1, **characterized in that** the several diluate spaces (16a, 16b, ...) and concentrate spaces (17a, 17b, ...) are operated in parallel and/or in series.

3. Method according to either of the preceding claims, **characterized in that** the diluate liquid, the concentrate liquid and the electrode washing solution, or one or other of these solutions, is/are temperature-controlled, preferably cooled, independently of each other.

4. Method according to one of the preceding claims, **characterized in that** the membranes have a pore size of from 1 to 1000 nm.

5. Method according to Claim 4, **characterized in that** the membranes are based on one of the following materials: cellulose ester, polyacrylonitrile, polyamide, polyether, polyethersulphone, polypropylene, polysulphone, polyvinyl alcohol or polyvinylidene fluoride or aluminium oxide, silicon oxide, titanium oxide or zirconium oxide, and also ceramics composed of the abovementioned oxides.

6. Method according to one of the preceding claims, **characterized in that** electrode washing solution flows through the anode space (21) and the cathode space (18) independently of each other.

7. Method according to one of the preceding claims, **characterized in that** the electrolytes employed for the diluate solution, the concentrate solution and the electrode washing solution contain a combination of weak acids and weak bases, weak acids and strong bases or strong acids and weak bases.

8. Method according to Claim 7, **characterized in that** the electrolytes contain one or more of the following compounds: boric acid, phosphoric acid, N-2-(acetamido)-2-aminoethanesulphonic acid, N-2-(acetamido)iminodiacetic acid, alanine, 2-amino-2-methyl-1,3-propanediol, ammonia, N,N-bis(2-hydroxyethyl)-2-aminoethanesulphonic acid, N,N-bis(2-hydroxyethyl)glycine, 2,2-bis(hydroxyethyl)iminotris(hydroxymethyl)metha ne, 2-cyclohexylamino(ethanesulphonic acid), acetic acid, glycine, glycylglycine, 2-[4-(2-hydroxyethyl)-1-piperazinyl]ethanesulphonic acid, 3-[4-(2-hydroxyethyl)-1-piperazinyl]propanesulphonic acid, histidine, imidazole, lactic acid, 2-morpholinoethanesulphonic acid, 2-morpholinopropanesulphonic acid, piperazine-1,4-bis(2-ethanesulphonic acid), N-[tris(hydroxymethyl)methyl]-2-aminoethanesulphonic acid, N-[tris(hydroxymethyl)methyl]glycine, triethanolamine, tris(hydroxymethyl)aminomethane and citric acid.

9. Method according to one of the preceding claims, **characterized in that** the current density, based on the membrane area, is from 10 to 1000 A/m$^2$, preferably from 10 to 500 A/m$^2$.

10. Method according to one of the preceding claims, **characterized in that** the conductivity of the diluate solution is from 0.1 mS/cm to 40 mS/cm, preferably from 0.1 to 10 mS/cm.

11. Method according to one of the preceding claims, **characterized in that**, after the separation, the diluate solution is concentrated, in particular by combining with a liquid permeation, such as microfiltration, ultrafiltration, nanofiltration or reverse osmosis, and returned to the diluate space (16).

12. Method according to one of the preceding claims, in which one or more of the following substances is/are treated: proteins, peptides, DNA, RNA, oligonucleotides, oligosaccharides, polysaccharides, viruses, virus constituents, cells, cell constituents, enantiomers and diastereomers.

13. Appliance for membrane electrophoresis, at least comprising a separation chamber (7a) which possesses two or more diluate spaces (16a, 16b, ...) and concentrate spaces (17a, 17b, ...) and also a cathode space (18) and an anode space (21) having electrodes as anode (19) and cathode (20), with the diluate spaces (16a, 16b, ...) and concentrate spaces (17a, 17b, ...) in each case being separated from each other by means of a separation membrane (15) and with cathode space (18) and anode space (21) being separated from the diluate spaces (16a, 16b, ...) and concentrate spaces (17a, 17b, ...), and also pairs of diluate spaces (16a, 16b, ...) and concentrate spaces (17a, 17b, ...) being separated within each other, by means of porous restriction membranes (14), feed lines (22) and discharge lines (23) for the diluate, feed lines (24) and discharge lines (25) for the concentrate, where appropriate feed lines (26) and discharge lines (27) for the electrode washing solution, and also a device (8; 10) or (9; 11) for pressure regulation by means of which a pressure difference of at least 3 kPa can be generated between diluate spaces (16a, 16b, ...) and concentrate spaces (17a, 17b, ...) and can be adjusted such that any liquid flow through the separation membranes (15) can essentially be prevented, with pressure-proof vessels (1, 2) having means for pressure superposition (12, 13) being provided as apparatus for pressure regulation.

14. Appliance according to Claim 13, **characterized in that** the several diluate spaces (16a, 16b, ...) and concentrate spaces (17a, 17b, ...)are connected in parallel and/or in series.

15. Appliance according to either of Claims 13 and 14, **characterized in that** feed lines (22) and discharge lines (23) for the diluate are arranged in a diluate circuit (1; 4; 22; 23), feed lines (24) and discharge lines (25) for the concentrate are arranged in a concentrate circuit (2; 5; 24; 25) and, where appropriate, feed lines (26) and discharge lines (27) for the electrode washing solution are arranged in an electrode washing circuit (3; 6; 26; 27).

16. Appliance according to one of Claims 13 to 15, **characterized in that** it possesses a diluate circuit, a concentrate circuit and an electrode washing circuit and heat exchangers in one or other or all of these circuits.

17. Appliance according to one of Claims 13 to 16, **characterized in that** porous membranes are provided with a pore size of from 1 to 1000 nm. They are preferably produced from organic or inorganic materials or from a mixture of the two.

18. Appliance according to one of Claims 13 to 17, **characterized in that** the membranes are based on one of the materials selected from the series: cellulose ester, polyacrylonitrile, polyamide, polyether, polyethersulphone, polypropylene, polysulphone, polyvinyl alcohol or polyvinylidene fluoride, or from aluminium oxide, silicon oxide, titanium oxide or zirconium oxide and also ceramics composed of the abovementioned oxides.

19. Appliance according to one of Claims 15 to 18, **characterized in that** the electrode washing circuit is constituted by a separate anode washing circuit and cathode washing circuit.

## Revendications

1. Procédé pour l'électrophorèse à membranes de substances dissoutes ou dispersées dans une solution contenant un électrolyte au moyen d'une chambre de séparation (7a), qui comporte plusieurs compartiments de diluat (16a, 16b,...), plusieurs compartiments de concentré (17a, 17b,...), ainsi qu'un compartiment cathodique (18) et un compartiment anodique (21) avec des électrodes sous forme d'anode (19) et de cathode (20), où les compartiments de diluat (16a, 16b,...) et les compartiments de concentré (17a, 17b,...) sont séparés à chaque fois les uns des autres par une membrane de séparation poreuse (15) et où le compartiment cathodique (18) et le compartiment anodique (21) sont séparés des compartiments de diluat (16a, 16b,...) et des compartiments de concentré (17a, 17b,...), de même que les paires de compartiments de diluat (16a, 16b,...) et de compartiments de concentré (17a, 17b,...) entre elles, par des membranes de restriction poreuses (14) et les électrodes (19, 20) sont baignées par une solution de rinçage des électrodes et le diluat est envoyé en continu dans le compartiment de diluat (16) et le concentré est envoyé en continu dans le compartiment de concentré (17), **caractérisé en ce qu'**au moins une substance dissoute ou dispersée dans le diluat est transférée par électrophorèse du compartiment de diluat (16) au compartiment de concentré (17) au moyen d'un champ électrique appliqué entre l'anode (19) et la cathode (20), où entre le compartiment de diluat (16) et le compartiment de concentré (17) est établie une différence de pression telle qu'un courant de liquide à travers la membrane de séparation (15) est sensiblement empêché et qui est d'au moins 3 kPa, et où cette différence de pression est régulée par superposition de pression dans les récipients de réserve (1, 2).

2. Procédé selon la revendication 1, **caractérisé en ce que** les différents compartiments de diluat (16a, 16b,...) et compartiments de concentré (17a, 17b,...) sont exploités en étant reliés en parallèle et/ou en série.

3. Procédé selon l'une des revendications précédentes, **caractérisé en ce que** le liquide de diluat, le liquide de concentré et la solution de rinçage des électrodes ou des solutions individuelles parmi ces solutions sont maintenus à température constante, de préférence sont refroidis, indépendamment les uns des autres.

4. Procédé selon l'une des revendications précédentes, **caractérisé en ce que** les membranes présentent une dimension de pores de 1 à 1 000 nm.

5. Procédé selon la revendication 4, **caractérisé en ce que** les membranes sont basées sur l'un des matériaux suivants : ester de cellulose, polyacrylonitrile, polyamide, polyéther, polyéthersulfone, polypropylène, polysulfone, poly(alcool vinylique), poly(fluorure de vinylidène) ou oxyde d'aluminium, oxyde de silicium, oxyde de titane, oxyde de zirconium ainsi que les céramiques mixtes des oxydes cités ci-dessus.

6. Procédé selon l'un des revendications précédentes, **caractérisé en ce que** le compartiment anodique (21) et le compartiment cathodique (18) sont rincés indépendamment l'un de l'autre avec la solution de rinçage des électrodes.

7. Procédé selon l'une des revendications précédentes, **caractérisé en ce que** les électrolytes utilisés pour la solution de diluat, la solution de concentré et la solution de rinçage des électrodes contiennent une combinaison d'acides faibles et de bases faibles, d'acides faibles et de bases fortes ou d'acides forts et de bases faibles.

8. Procédé selon la revendication 7, **caractérisé en ce que** les électrolytes contiennent un ou plusieurs des composés suivants : acide borique, acide phosphorique, acide N-2-(acétamido)-2-aminoéthanesulfonique, acide N-2-(acétamido)iminodiacétique, alanine, 2-amino-2-méthyl-1,3-propanediol, ammoniac, acide N,N-bis(2-hydroxyéthyl)-2-aminoéthanesulfonique, N,N-bis-(2-hydroxyéthyl)glycine, 2,2-bis(hydroxyéthyl)iminotris(hydroxyméthyl)méthane, acide 2-(cyclohexylamino)éthanesulfonique, acide acétique, glycine, glycylglycine, acide 2-[4-(2-hydroxyéthyl)-1-pipérazinyl]éthanesulfonique, acide 3-[4-(2-hydroxyéthyl)-1-pipérazinyl]propanesulfonique, histidine, imidazole, acide lactique, acide 2-morpholinoéthanesulfonique, acide 2-morpholinopropanesulfonique, acide pipérazine-1,4-bis(2-éthanesulfonique), acide N-[tris(hydroxyméthyl)méthyl]-2-aminoéthanesulfonique, N-[tris(hydroxyméthyl)méthyl]-glycine, triéthanolamine, tris(hydroxyméthyl)aminométhane, acide citrique.

9. Procédé selon l'une des revendications précédentes, **caractérisé en ce que** la densité de courant rapportée à la surface des membranes est 10 à 1 000 A/m$^2$, de préférence 10 à 500 A/m$^2$.

10. Procédé selon l'une des revendications précédentes, **caractérisé en ce que** la conductivité de la solution de diluat est 0,1 mS/cm à 40 mS/cm, de préférence 0,1 à 10 mS/cm.

11. Procédé selon l'une des revendications précédentes, **caractérisé en ce que** la solution de diluat est concentrée à la suite de la séparation, en particulier par combinaison avec une perméation de liquide, comme par exemple une microfiltration, une ultrafiltration, une nanofiltration ou une osmose inverse et est renvoyée dans le compartiment de diluat (16).

12. Procédé selon l'une des revendications précédentes, où une ou plusieurs des substances suivantes sont traitées : protéines, peptides, ADN, ARN, oligonucléotides, oligo- et polysaccharides, virus, constituants de virus, cellules, constituants de cellules, énantiomères et diastéréoisomères.

13. Dispositif pour l'électrophorèse à membranes comportant au moins une chambre de séparation (7a), qui comporte plusieurs compartiments de diluat (16a, 16b,...) et plusieurs compartiments de concentré (17a, 17b,...) ainsi qu'un compartiment cathodique (18) et un compartiment anodique (21) avec des électrodes sous forme d'anode (19) et de cathode (20), où les compartiments de diluat (16a, 16b,...) et les compartiments de concentré (17a, 17b,...) sont séparés les uns des autres par une membrane de séparation (15) et où le compartiment cathodique (18) et le compartiment anodique (21) sont séparés des compartiments de diluat (16a, 16b,...) et des compartiments de concentré (17a, 17b,...), de même que les paires de compartiments de diluat (16a, 16b,...) et de compartiments de concentré (17a, 17b,...) entre elles, par des membranes de restriction poreuses (14), des conduits d'amenée (22) et des conduits d'évacuation (23) pour le diluat, des conduits d'amenée (24) et des conduits d'évacuation (25) pour le concentré, éventuellement des conduits d'amenée (26) et des conduits d'évacuation (27) pour la solution de rinçage des électrodes ainsi qu'un dispositif (8 ; 10) ou (9 ; 11) pour la régulation de la pression, au moyen duquel une différence de pression d'au moins 3 kPa peut être établie entre les compartiments de diluat (16a, 16b,...) et les compartiments de concentré (17a, 17b,...) et peut être réglée de telle manière qu'un courant de liquide à travers les membranes de séparation (15) peut être sensiblement empêché, où des récipients de réserve étanches à la pression (1, 2) avec des moyens pour la superposition de pression (12, 13) sont prévus comme dispositif pour la régulation de la pression.

14. Dispositif selon la revendication 13, **caractérisé en ce que** les différents compartiments de diluat (16a, 16b,...) et compartiments de concentré (17a, 17b,...) sont branchés en parallèle et/ou en série.

15. Dispositif selon l'une des revendications 13 ou 14, **caractérisé en ce que** les conduits d'amenée (22) et les conduits d'évacuation (23) pour le diluat sont disposés dans un circuit de diluat (1 ; 4 ; 22 ; 23), les conduits d'amenée (24) et les conduits d'évacuation (25) pour le concentré sont disposés dans un circuit de concentré (2 ; 5 ; 24 ; 25), éventuellement les conduits d'amenée (26) et les conduits d'évacuation (27) pour la solution de rinçage des électrodes sont disposés dans un circuit de rinçage des électrodes (3 ; 6 ; 26 ; 27).

16. Dispositif selon l'une des revendications 13 à 15, **caractérisé en ce qu'**il comporte un circuit de diluat, un circuit de concentré et un circuit de rinçage des électrodes et **en ce qu'**il comporte des échangeurs de chaleur dans des circuits individuels ou dans tous ces circuits.

17. Dispositif selon l'une des revendications 13 à 16, **caractérisé en ce que** des membranes poreuses ayant une dimension de pores de 1 à 1 000 nm sont prévues. Celles-ci sont de préférence produites à partir de matériaux organiques ou inorganiques ou d'un mélange des deux.

18. Dispositif selon l'une des revendications 13 à 17, **caractérisé en ce que** les membranes sont basées sur l'un des matériaux choisis dans la série :

ester de cellulose, polyacrylonitrile, polyamide, polyéther, polyéthersulfone, polypropylène, polysulfone, poly (alcool vinylique), poly(fluorure de vinylidène) ou oxyde d'aluminium, oxyde de silicium, oxyde de titane, oxyde de zirconium ainsi que les céramiques mixtes des oxydes cités ci-dessus.

19. Dispositif selon l'une des revendications 15 à 18, **caractérisé en ce que** le circuit de rinçage des électrodes est constitué par un circuit de rinçage d'anode et un circuit de rinçage de cathode séparés.

Abb. 1: Schema einer Membranelektrophorese-Anlage mit Einrichtung zur Drucküberlagerung

Abb. 2: Schema eines Membranelektrophorese-Moduls (Vierer-Stack)

**EP 1 572 329 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3989613 A
- US 4043895 A
- DE 3337669 A1
- DE 3626953 A1
- US 6270672 A
- US 4243507 A

**In der Beschreibung aufgeführte Nicht-Patentliteratur**

- **Galier et al.** *J. Membr. Sci.,* 2001, vol. 194, 117-133